(19)

Europäisches
Patentamt
European
Patent Office
Office européen
des brevets

(11) **EP 2 085 080 A1**

(12) **EUROPEAN PATENT APPLICATION**

(43) Date of publication:
**05.08.2009 Bulletin 2009/32**

(51) Int Cl.:
***A61K 9/51*** *(2006.01)*

(21) Application number: **08150853.3**

(22) Date of filing: **30.01.2008**

(84) Designated Contracting States:
**AT BE BG CH CY CZ DE DK EE ES FI FR GB GR
HR HU IE IS IT LI LT LU LV MC MT NL NO PL PT
RO SE SI SK TR**
Designated Extension States:
**AL BA MK RS**

(71) Applicant: **LEK Pharmaceuticals d.d.**
**1526 Ljubljana (SI)**

(72) Inventor: **The designation of the inventor has not
yet been filed**

(74) Representative: **Kröger, Bernd et al
Sandoz International GmbH
Global Patents Department
Industriestrasse 25
83607 Holzkirchen (DE)**

(54) **Preparation of nanoparticles by using a vibrating nozzle device**

(57)    A method for preparing nanoparticles is provided, which comprises the steps of dissolving a polymer and, optionally, at least one additional ingredient, in an organic solvent, passing the solution through a vibrating nozzle and dropping the solution into an aqueous solution, which is stirred, such that nanoparticles are formed by the rapid diffusion of the solvent.

EP 2 085 080 A1

**Description**

The production of small particles is important in many technical fields including but not limited to pharmaceutics, nutraceutics, food processing, paints and copying technologies. In general, smaller particles sizes may lead to the development of new products as well as to more effective products. New techniques for generating particles with decreased particle size are necessary for future development in many scientific and industrial fields.

The present invention relates to a method for a simple, reproducible and economical manufacturing of polymer nanoparticles. In particular, the present invention relates to a method for preparing nanoparticles with a nanoprecipitation method using a vibrating nozzle device. This automated technique presents some advantages such as high reproducibility, the possibility to scale up the production and the possibility for producing sterile nanoparticles.

Ingredients which can be entrapped in the nanoparticles or adsorbed at the surface of the nanoparticles include any ingredient which it may be desired to administer to the human body for any purpose, including therapeutic, diagnostic, cosmetic and prophylactic agents. The additional ingredients may be ingredients for veterinary and agricultural use, food products or additives, colorants or any other ingredients.

At present, various techniques are known for preparing aqueous nanoparticle dispersions. These techniques include emulsification-evaporation, nanoprecipitation, salting-out and emulsification-diffusion.

The nanoprecipitation technique is a known technique for the preparation of nanoparticles. In this method, a polymer, a drug and, optionally, a lipophilic stabilizer (e.g. phospholipids) are dissolved in a semipolar water-miscible solvent, such as acetone or ethanol. This solution is poured or injected into an aqueous solution containing a stabilizer (e.g. polyvinyl alcohol (PVAL) or poloxamer 188) under magnetic stirring. During this procedure, nanoparticles are formed instantaneously by the rapid diffusion of the solvent, which is then eliminated from the suspension under reduced pressure.

In order to obtain a particle size in the nanometric range a good distribution of organic phase into aqueous phase is crucial. In laboratory scale this can be achieved by agitation of the aqueous phase during the addition of an organic polymer solution. However, with enlargement of the volume this method becomes ineffective. Therefore, hitherto the nanoparticle production by nanoprecipitation is limited to small quantities and requires a lot of time. Accordingly, until the present invention a scale up of the nanoprecipitation method was difficult.

US Patent No. 5,118,528 discloses the original method of nanoprecipitation. The method according to this document comprises: (1) the preparation of a liquid phase consisting essentially of a solution of the substance in a solvent or in a mixture of solvents to which may be added one or more surfactants, (2) the preparation of a second liquid phase consisting essentially of a non-solvent or a mixture of non-solvents for the substance and to which may be added one or more surfactants, the non-solvent or the mixture of non-solvents for the substance being miscible in all proportions with the solvent or the mixture of solvents for the substance, (3) the addition of one of the liquid phases prepared in (1) or (2) to the other with moderate stirring so as to produce a colloidal suspension of nanoparticles of the substance, and (4) is desired, the removal of all or part of the solvent or the mixture of solvents for the substance and of the non-solvent or the mixture of non-solvents for the substance so as to produce a colloidal suspension of nanoparticles of the desired concentration or to produce a powder of nanoparticles.

EP-A-1 728 814 discloses a method for forming particles using a porous membrane. According to this document, a solvent with the dissolved polymer is pumped from a solvent container and a flocculating agent is pumped from a flocculating agent container in two circulations and in two volumes divided by the porous membrane of a membrane reactor. The precipitated nanoparticles with size of 60 nm are filtered in a filtration step.

EP-A-0 235 603 discloses a method for forming uniform liquid droplets having a particle size of not more than 250 $\mu$m which comprises jetting a liquid having a viscosity of 50 to 2000 cP through at least one orifice having a diameter of 20 to 100 $\mu$m, while directly vibrating said liquid at a frequency of 300 to 40000 Hz with a vibrating rod. The frequency capable of giving the uniform liquid droplets is decided depending on the orifice diameter, the flow rate, the viscosity and surface tension of the liquid or the amplitude of the vibrating rod, but does not depend on the composition of the liquid. The method is aplicable, for instance, to a solution of a natural or syntetic high polymer, and when liquid droplets formed from such a solution are solidified, spherical solid particles having a uniform particle size are obtained.

US 2004/0022939 A1 and US Patent No. 6,669,961, respectively, disclose a method of forming microparticles, which comprises accelerating a stream comprising a liquid and vibrating the stream, to form particles. The particle may have a diameter that is smaller than the diameter of the nozzle used to form the stream, allowing for the formation of micro- and nanosized particle. According to this method, the addition of an organic phase to the aqueous phase should be controlled and constant, by mild stirring, to assure an uniform distribution and diffusion.

Fessi et al., 1989 H. Fessi, F. Puisieux, J.P. Devissaguet, N. Ammoury and S. Benita, Nanocapsule formation by interfacial polymer deposition following solvent displacement, Int. J. Pharm. 55 (1989), pp. 25-28; describes a technological procedure for the production of nanocapsules.

Furthermore, there are several publications directed to methods for producing micro- and nanoparticles:

Prakobvaitayakit and Nimmannit (AAPS PharmSciTech, 4(4), 1-9 (2003)) used a constant flow rate of 0.3 ml/min with mechanical stirring of 750 rpm. In the work of Govender et al. (Journal of Controlled Release, 57, 171-185 (1999)) a dropwise organic phase addition has been reported. The stirring was done by a magnetic stirrer. The same procedure was followed by Saxena et al. (Int. J. Pharm, 278 (2), 293-301 (2004)). Csaba et al. (Journal of Biomaterials Science, Polymer edition, 15 (9), 1137-1151 (2004); Biomacromolecules, 6, 271-278 (2005)) used vortex agitation for mixing both phases getting a fast organic phase dispersion and further moderate magnetic stirring. Other works using fast organic phase dispersion are the publications of Ameller et al. (European Journal of Pharmaceutical Sciences, 21, 361-370 (2004); Pharmaceutical Research, 20(7), 1063-1070 (2003))

The term "nanoparticles" (NP) as used in the present invention is directed to particles having a diameter ≤500nm. The nanoparticles according to the present invention are nanospheres.

The present method for the preparation of nanoparticles with a nanoprecipitation method using vibrating nozzle device (e.g. Encapsulator Inotech IE-50R, Inotech, Swiss) is automated technique and presents some advantages such as high reproducibility and the possibilities of scale up and preparation of sterile nanoparticles. According to the present invention the preparation of the nanoparticles may be continuous or non continuous.

The present invention is based on the idea that passing or pumping an organic phase through a nozzle and splitting this jet in very small droplets enables a better distribution of organic phase in an aqueous phase in comparison with injecting, leading to the formation of very small and homogeneous nanoparticles.

In the following, preferred embodiments of the invention are described.

In a method according to the present invention, nanoparticles may be prepared in the following way:

A polymer and, optionally, at least one additional ingredient, preferably a drug, is/are dissolved in an organic solvent (organic phase; e.g. acetone), passed, preferably pumped, through a vibrating nozzle and are dropped into an aqueous solution, which preferably comprises a stabilizer (e.g. an aqueous solution of polyvinyl alcohol), which aqueous solution is stirred (e.g. magnetically). A colloidal dispersion comprising nanoparticles is formed instantaneously by the rapid diffusion of the solvent, which may be then eliminated from the suspension by evaporating under atmospheric pressure at room temperature or under reduced pressure.

Biocompatible and biodegradable polylactides/glycolides (PLA/PLGA) have received high attention over the last thirty years in the biomedical field as sutures, implants, colloidal drug delivery systems, and more recently also in tissue repairing and engineering and anti-cancer drug delivery.

Accordingly, a matrix polymer which may be used in the present invention is preferably selected from biodegradable polymers such as polylactide (PLA), polyglycolide (PGA), copolymers of lactide and glycolide (PLGA), poly($\varepsilon$-caprolactone) (PCL).

However, depending on the intended use of the nanoparticles, it is also possible to use non-biodegradable polymers such as copolymers of acrylic and methacrylic acid esters, cellulose acetate phthalate (CAP), cellulose acetate trimellitate (CAT), and ethylene vinyl acetate copolymer (EVAC).

It is preferred that the polymer is present in the organic solvent (organic phase) in a concentration range of 0,1 to 10 % w/w.

Water-miscible organic solvents which may be used in the present invention are prefereably selected on the basis of their volatility and low toxicity, in particular when a pharmaceutical application for the resulting aqueous colloidal dispersion is considered. The "solvent" or the mixture of solvents used is a liquid capable of dissolving the polymer and the drug. Moreover, the solvent is preferably miscible with the non-solvent (aqueous phase, optionally with a stabilizer) for the polymer and the drug used in the preparation and sufficiently volatile in order to be removable after the product preparation. The solvent may preferably be chosen from among a lower alcohol (methanol, ethanol, isopropanol, etc.), a lower ketone (acetone, methylethyl-ketone, 2-butanone, etc.), esters (ethyl acetate, methyl acetate, isopropyl acetate) or other common solvents such as acetonitrile, etc. Particularly preferred are acetone and ethanol because of their widely recognized low toxicity, good solubilizing properties and low boiling points.

The viscosity of organic phase comprising the polymer and optionally a further substance, is preferably in the range of 0,3 to 1 cP, and the surface tension is preferably in the range of 20 to 25 mN/m.

The method for the preparation of the nanoparticles is preferably conducted at room temperature (i.e. 20 to 25 °C) and a relative humidity of 40 to 60 %.

As described above, stabilisers or stabilizing agents of the hydrophilic phase (nonsolvent, aqueous solution) are preferably used in the present invention. These stabilizing agents are not specifically restricted as long as they are able to stabilize the hydrophilic phase. Stabilizing agents, which are particularly preferred for stabilizing the final dispersion are poly (vinyl alcohol) (PVAL) and poloxamers or mixtures thereof, because of their good water solubility, suitability for ingestion, lyoprotective properties and compatibility with the system. The stabilizing agent may be contained in the aqueous phase preferably in an amount of not more than 1 % w/w, and more preferably in an amount of not more than 0,1 % w/w, on the basis of the initial aqueous solution.

As described above, stabilisers or stabilizing agents of the lipophilic phase (organic solvent) are preferably used in the present invention. These stabilizing agents are not specifically restricted as long as they are able to stabilize the lipophilic phase. Suitable stabilisers of the lipophilic phase, which may be used in the method of the present invention, preferably include phospholipids, sorbitan esters (such as Span 60 - sorbitan monostearate), glyceryl monoesters (such as glyceryl monostearate), and nonyl phenol ethoxylates or any other stabiliser which is soluble in the organic solvent. The concentration of the lipophilic stabilizer is preferably ≤ 1 % (w/w) on the basis of the organic solvent.

The resulting colloidal suspension of nanoparticles may comprise 0,05 to 10 % w/w of at least one surfactant.

The delivery system prepared according to the method of the invention are particularly well suited for use with active compounds such as pharmacologically active proteins, peptides, vaccines, and the like, as well as with other small pharmacologically active molecules and contrast agents. Specific active compounds include celecoxib, budesonide, paclitaxel, camptothecin, 9-nitrocamptothecin, cisplatin, carboplatin, ciprofloxacin, doxorubicin, rolipram, simvastatin, methotrexate, indomethacin, probiprofen, ketoprofen, iroxicam, diclofenac, cyclosporine, etraconazole, rapamycin, nocodazole, colchicine, ketoconazole, tetracycline, minocycline, doxycycline, ofloxacin, gentamicin, octreotide, calcitonin, interferon, testosterone, progesterone, estradiol, estrogen, and insulin.

In the method of the present invention, preferably an encapsulator device is used. The core of the encapsulator nozzle may be a precisely drilled sapphire disc. Custom nozzles are available in aperture sizes of 50 to 1000 $\mu$m. Preferably, for the preparation of nanoparticles a 500 $\mu$m nozzle is used.

In a preferred embodiment of the present invention the method is conducted by using the following conditions:

The vibration control system generates an appropriate electric oscillation for an electromagnetic coil. Nanoparticles are preferably produced in the frequency range between 50 and 7000 Hz, more preferably at 450 and 550 Hz.

The amplitude of the nozzle vibration may be between 1 and 7. For good nanoparticles production values between 1 and 4 are preferred.

The preferred flow rate for organic solvent through the device and the nozzle is from 8 to 10 ml/min.

A feature of the Encapsulator which is preferably used in this is the ability to charge the surface of the droplets. The repulsion forces induced by the equally charged surfaces prevent the droplets from hitting each other in flight, and from hitting each other as they enter the nonsolvent (aqueous phase). The applied voltage may be in the range from 0 to 1800 V, preferably from 800 to 1000 V.

As mentioned above, the method for the preparation of nanoparticles by using a vibrating nozzle device has advantages such as automatization, high reproducibility, the possibility of scale up and the possibility of production of sterile nanoparticles. The preparation of the nanoparticles may be continuous or non continuous.

**Examples**

The present invention is illustrated but in no way limited by the following examples, wherein the following materials, conditions and measuring methods were used:

*Freeze drying of nanoparticle dispersions*

Celecoxib loaded PLA/PLGA nanoparticle dispersions were freeze-dried. Samples were frozen at - 70 °C and freeze dried at 0.01 bar at room temperature.

*Particle size and zeta potential analysis*

The mean particle size and polydispersity index were estimated by a photon correlation spectroscopy (PCS) using a Zetasizer 3000 (Malvern, UK). The analysis was performed at a scattering angle of 90 ° and at a temperature of 25 °C. Before measuring samples were diluted with dust-free water to give the recommended scattering intensity. The diameter was calculated from the autocorrelation function of the intensity of light scattered from particles, assuming a spherical form for the particles. The polydispersity index (PI) is a measure of dispersion homogeneity and ranges from 0 to 1. Values close to 0 indicate a homogeneous dispersion while those greater than 0.3 indicate high heterogeneity.

The particle charge was quantified as zeta potential by laser Doppler anemometry using a Zetasizer 3000. The samples were diluted with distilled water adjusted to a conductivity of 50 $\mu$S/cm$^2$ with a solution of 0,9 % NaCl. The zeta potentials was calculated by the Helmholtz-Smoluchowski equation.

*Determination of drug content in the nanoparticles*

The amount of celecoxib encapsulated per unit weight of NP was determined dissolving a weight amount of freeze dried NP in acetonitrile : water (1 : 1, v/v) mixture, filtered through 0,45 $\mu$m siringe filter in order to remove the precipitated PVA and than measuring the amount of the drug by an HPLC method using a Hypersil ODS C-18 column (5 $\mu$m, 300 x 3,9 mm; BIA Separations d.o.o. Ljubljana, Slovenia). The mobile phase was a mixture of acetonitrile, phosphate buffer (pH 4.0, 0.01 M) and methanol at a ratio of 45:45:10 (v/v). The detection wavelength was 238 nm and the flow rate 1,4 ml/min. The retention time under these conditions was 10,5 min. Drug content and entrapment efficiency (EE) were calculated according to Eqs. (1) and (2), respectively. The mean value of three replicate determinations is reported.

$$\text{Drug content (\% w/w)}$$

$$= \frac{\text{mass of drug in nanoparticles} \times 100}{\text{mass of nanoparticles}}$$

$$\text{Entrapment efficiency (\% w/w)}$$

$$= \frac{\text{mass of drug in nanoparticles} \times 100}{\text{total mass of drug used in preparation of nanoparticles}}$$

PLA and D,L-PLGA 50/50 and 75/25 (lactic acid/glycolic acid) copolymers (Resomer 202H, RG 502 and 752 respectively) were purchased from Boehringer (Ingelheim, Germany). Polyvinyl alcohol (PVA) having a molecular weight of 27000 g/mol (Mowiol 4-98, Hoechst AG, Germany) was chosen as a stabilizing hydrocolloid. Acetone and acetonitrile were purchased from Merck (Germany). All other chemicals and solvents were of reagent grade and used as received.

Table: PLA/PLGA ratio and molecular weight of different polymers

| Type of polymer | PLA/PLGA ratio | MW [Da] |
|---|---|---|
| Resomer RG 502 | 50/50 | 14000 |
| Resomer RG 752 | 75/25 | 20000 |
| Resomer R 202 H | 100/0 | 17000 |

In the Examples an Encapsulator (Inotech IE-50 R, Inotech, Swiss) has been used as the vibrating nozzle device, which was originally used for formation of pellets and microcapsules.

The syringe pump which may be used in this embodiment was regulated with the syringe pumpe control system, that generates the selected elecrical signal. On pumping speed display it could be indicated a number between 0 and 950, which is correlated to the syringe pump speed. Used value 353 corresponds to flow rate around 9 ml/min.

The preparation of NP was achieved by adjusting the nanoprecipitation technique, previously applied to the preparation of NP (Fessi et al. 1989).

EXAMPLE 1

*a) Preparation of nanoparticles (NP)*

550 mg of polylactic acid (PLA) / poly(lactic-co-glycolic acid) (PLGA) were dissolved in 15 ml of acetone and the solution was passed through a 500 nm nozzle of the encapsulator and allowed to drop into 200 ml of an aqueous polyvinyl alcohol solution (0.1-0.9 % w/w) which was stirred magnetically. The experiments were performed at a flow rate (stream velocity) of 8.8 ml/min. The vibration frequency used to break up the liquid jet was set at 482 Hz and the amplitude at 3.5. Afterwards, the organic solvent was evaporated under atmospheric pressure at room temperature and the aggregates were removed by filtration.

*b)Characterization of prepared nanoparticles*

Table 1 Effect of type of polymer and initial drug : polymer ratio on the mean particle diameter (d) and polydispersity index (PI) of nanoparticles. PVA concentration = 0.45 %, n = 3 (the experiment was done in triplicate).

Table 1

| Type of polymer | Initial ratio drug:polymer | d [nm] | PI |
|---|---|---|---|
| RG 502 | 1:5 | 224.0 +/- 10.6 | 0.15 +/- 0.01 |
| | 1:10 | 223.6 +/- 6.0 | 0.15 +/- 0.02 |
| | 1:20 | 225.6 +/- 1.6 | 0.11 +/- 0.05 |
| RG 752 | 1:10 | 269.4 +/- 3.4 | 0.20 +/- 0.01 |
| R 202 H | 1:10 | 242.4 +/- 5.6 | 0.24 +/- 0.01 |

Table 2 Effect of stabilizer concentration on the mean particle diameter (d) and the polydispersity index (PI) of nanoparticles. Polymer =Resomer RG 502, initial drug : polymer ratio = 1:10, n =3.

Table 2

| PVA (% w/V) | d [nm] | PI |
|---|---|---|
| 0.900 | 227.4 +/- 2.0 | 0.19 +/- 0.01 |
| 0.675 | 224.0 +/- 6.4 | 0.15 +/- 0.02 |
| 0.450 | 223.6 +/- 6.0 | 0.15 +/- 0.02 |
| 0.225 | 223.4 +/- 2.2 | 0.11 +/- 0.04 |
| 0.100 | 226.9 +/- 5.9 | 0.10 +/- 0.05 |

It can be seen from the above results that PLA and PLGA nanoparticles having a diameter below 250 nm can be readily prepared with good reproducibility by the present nanoprecipitation method using a vibrating nozzle device.

EXAMPLE 2

*Encapsulation of drugs*

The ability of the inventive method to incorporate drugs within the polymer nanoparticles is demonstrated using celecoxib (4-[5-(4-methylphenyl)-3-(trifluoromethyl)pyrazol-1-yl]benzenesulfonamide) as a drug. The substance celecoxib (27.5, 55 and 110 mg) was dissolved in a PLGA/acetone solution. Nanoparticles were fabricated using the procedures described in Example 1.

Table 3 Effect of type of polymer and initial drug : polymer ratio on entrapment efficiency (EE) and drug content of celecoxib in nanoparticles. PVA concentration = 0.45 %, n = 3.

Table 3

| Type of polymer | Initial ratio drug:polymer | % EE | drug content [%] |
|---|---|---|---|
| RG 502 | 1:5 | 83.9 +/- 1.7 | - |
| | 1:10 | 89.2 +/- 5.4 | 3.9 +/- 0.2 |
| | 1:20 | 98.6 +/- 1.4 | - |
| RG 752 | 1:10 | 70.8 +/- 7.2 | 3.4 +/- 0.6 |
| R 202 H | 1:10 | 71.0 +/- 6.9 | 3.4 +/- 0.3 |

Table 4 Effect of stabilizer concentration on entrapment efficiency (EE) and drug content of celecoxib in nanoparticles. Polymer =Resomer RG 502, initial drug : polymer ratio = 1:10, n =3.

Table 4

| PVA (% w/V) | % EE | drug content [%] |
|---|---|---|
| 0.900 | - | - |
| 0.675 | - | - |
| 0.450 | 89.2 +/- 5.4 | 3.9 +/- 0.2 |
| 0.225 | 84.0 +/- 3.0 | 5.2 +/- 0.5 |
| 0.100 | 82.2 +/- 4.2 | 5.9 +/- 0.1 |

Table 5 Effect of stabilizer (PVA) concentration and initial drug : polymer Resomer RG 502 ratio on drug content of celecoxib in nanoparticles. n =3.

Table

| % PVA in water phase | drug content [%] | |
|---|---|---|
| | drug/polymer = 1:5 | drug/polymer = 1:10 |
| 0.45 | 6.8 +/- 0.1 | 3.9 +/- 0.2 |
| 0.225 | 9.7 +/- 0.7 | 5.2 +/- 0.5 |
| 0.1 | 13.0 +/- 2.5 | 5.9 +/- 0.1 |

The highest drug content (13 %) in nanoparticles was obtained by using 0.1 % PVA solution as stabilizer and the initial ratio celecoxib : Resomer RG 502 = 1:5.

**Claims**

1. A method for preparing nanoparticles, comprising the steps of :

    dissolving a polymer and, optionally, at least one additional ingredient in an organic solvent,
    passing the solution through a vibrating nozzle, and
    dropping the solution into an aqueous solution, which is stirred, such that nanoparticles are formed by the rapid diffusion of the solvent.

2. The method according to claim 1, further comprising the step of evaporating the solvent from the suspension under atmospheric pressure at room temperature or under reduced pressure.

3. The method according to claim 1 or 2, wherein the additional ingredient is a drug.

4. The method according to claim 3, wherein the drug is selected from the group consisting of pharmacologically active proteins, peptides, vaccines, celecoxib, budesonide, paclitaxel, camptothecin, 9-nitrocamptothecin, cisplatin, carboplatin, ciprofloxacin, doxorubicin, rolipram, simvastatin, methotrexate, indomethacin, probiprofen, ketoprofen, iroxicam, diclofenac, cyclosporine, etraconazole, rapamycin, nocodazole, colchicine, ketoconazole, tetracycline, minocycline, doxycycline, ofloxacin, gentamicin, octreotide, calcitonin, interferon, testosterone, progesterone, estradiol, estrogen, and insulin, or mixtures thereof.

5. The method according to any one of the preceding claims, wherein the aqueous solution comprises a stabilizer.

6. The method according to claim 5, wherein the stabilizer is selected from the group consisting of polyvinyl alcohol and poloxamers, or mixtures thereof.

7. The method according to claim 5 or 6, wherein the concentration of the stabilizer in the aqueous solution is ≤ 1 % (w/w).

8. The method according to claim 7, wherein the concentration of the stabilizer in the aqueous solution is ≤ 0.1 % (w/w).

9. The method according to any one of the preceding claims, wherein the organic solvent comprises a lipophilic stabilizer.

10. The method according to claim 9, wherein the lipophilic stabilizer is selected from the group consisting of phospholipids, sorbitan esters, glyceryl monoesters and nonyl phenol ethoxylates or mixtures thereof.

11. The method according to claim 9 or 10, wherein the concentration of the lipophilic stabilizer in the organic solvent is ≤ 1 % (w/w).

12. The method according to any one of the preceding claims, wherein the organic solvent is selected from the group consisting of lower alcohol, a lower ketone, esters, acetonitrile or mixtures thereof.

13. The method according to claim 12, wherein the organic solvent is acetone and/or ethanol.

14. The method according to any one of the preceding claims, wherein the polymer is selected from the group consisting of biodegradable polymers, preferably polylactide (PLA), polyglycolide (PGA), copolymers of lactide and glycolide (PLGA), poly(ε- caprolactone) (PCL) and non-biodegradable polymers, preferably copolymers of acrylic and methacrylic acid esters, cellulose acetate phthalate (CAP), cellulose acetate trimellitate (CAT), and ethylene vinyl acetate copolymer (EVAC), or mixtures thereof.

15. The method according to any one of the preceding claims, wherein the concentration of the polymer in the organic solvent is in the range from 0,1 to 10 % w/w.

16. The method according to any one of the preceding claims, wherein the frequency of the vibration is in the range from 50 to 7000 Hz.

17. The method according to claim 11, wherein the frequency of the vibration is in the range from 450 to 550 Hz.

18. The method according to any one of the preceding claims, wherein the amplitude of the vibration is in the range from 1 to 7.

19. The method according to claim 12, wherein the amplitude of the vibration is in the range from 1 to 4.

20. The method according to any one of the preceding claims, wherein the flow rate of the solution passed through the nozzle is in the range from 8 to 10 ml/min.

21. The method according to any one of the preceding claims, wherein an voltage in the range from 0 to 1800 V, preferably from 800 to 1000 V, is applied to the droplets.

22. The method according to any one of the preceding claims, wherein the method is conducted under sterile conditions.

**European Patent Office**

# EUROPEAN SEARCH REPORT

Application Number

EP 08 15 0853

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2005/072709 A (BIO DAR LTD [IL]; YISSUM RES DEV CO [IL]; MAGDASSI SHLOMO [IL]; SELA Y) 11 August 2005 (2005-08-11)<br>* page 5, lines 1-4 *<br>* page 9, line 3 - page 10, line 2 *<br>* page 10, last paragraph - page 12, paragraph 1 *<br>* examples 1-6 *<br>* page 6, last paragraph - page 7, last paragraph *<br>----- | 1-22 | INV.<br>A61K9/51 |
| D,X | US 2002/054912 A1 (KIM KYEKYOON [US] ET AL) 9 May 2002 (2002-05-09)<br>* figure 7 *<br>* paragraph [0017] - paragraph [0022] *<br>* paragraph [0066] - paragraph [0067] *<br>* paragraph [0077] - paragraph [0080] *<br>* paragraph [0093] - paragraph [0095] *<br>* paragraph [0115] *<br>* examples 1-3 *<br>----- | 1-22 | |
| D,A | EP 0 235 603 A (KANEGAFUCHI CHEMICAL IND [JP]) 9 September 1987 (1987-09-09)<br>* page 8, line 8 - line 16 *<br>* examples 1-11 *<br>* tables 2,3 *<br>* claims 1-3 *<br>----- | 1-22 | TECHNICAL FIELDS SEARCHED (IPC)<br><br>A61K |

The present search report has been drawn up for all claims

| Place of search | Date of completion of the search | Examiner |
|---|---|---|
| The Hague | 26 May 2008 | S. von Eggelkraut-G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 08 15 0853

This annex lists the patent family members relating to the patent documents cited in the above-mentioned European search report.
The members are as contained in the European Patent Office EDP file on
The European Patent Office is in no way liable for these particulars which are merely given for the purpose of information.

26-05-2008

| Patent document cited in search report | | Publication date | Patent family member(s) | | Publication date |
|---|---|---|---|---|---|
| WO 2005072709 | A | 11-08-2005 | EP | 1708682 A2 | 11-10-2006 |
| | | | US | 2008038333 A1 | 14-02-2008 |
| US 2002054912 | A1 | 09-05-2002 | US | 2004022939 A1 | 05-02-2004 |
| EP 0235603 | A | 09-09-1987 | AU | 6850287 A | 13-08-1987 |
| | | | JP | 2116135 C | 06-12-1996 |
| | | | JP | 6020528 B | 23-03-1994 |
| | | | JP | 62191033 A | 21-08-1987 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5118528 A
- EP 1728814 A
- EP 0235603 A
- US 20040022939 A1
- US 6669961 B

**Non-patent literature cited in the description**

- **H. Fessi ; F. Puisieux ; J.P. Devissaguet ; N. Ammoury ; S. Benita.** Nanocapsule formation by interfacial polymer deposition following solvent displacement. *Int. J. Pharm.,* 1989, vol. 55, 25-28
- *AAPS PharmSciTech,* 2003, vol. 4 (4), 1-9
- **Govender et al.** *Journal of Controlled Release,* 1999, vol. 57, 171-185
- **Saxena et al.** *Int. J. Pharm,* 2004, vol. 278 (2), 293-301
- **Csaba et al.** Journal of Biomaterials Science. 2004, vol. 15, 1137-1151
- *Biomacromolecules,* 2005, vol. 6, 271-278
- **Ameller et al.** *European Journal of Pharmaceutical Sciences,* 2004, vol. 21, 361-370
- *Pharmaceutical Research,* 2003, vol. 20 (7), 1063-1070